# EUROPÄISCHE PATENTSCHRIFT

(11) **EP 1 203 570 B1**
(45) Veröffentlichungstag und Bekanntmachung des Hinweises auf die Patenterteilung: **08.03.2006**
(21) Anmeldenummer: 01126209.4
(22) Anmeldetag: 05.11.2001
(51) Int. Cl.: A61F 5/56

(54) **Aufbissschiene zur Verhinderung von Schnarchen und von obstruktiver Schlafapnoe sowie zur künstlichen Beatmung**
Gumshield for preventing snoring and obstructiv apnea and for artificial respiration
Appareil dentaire destiné à la prevention de ronflement, de l'apnée pendant le sommeil et à la respiration artificielle

(30) Priorität: 03.11.2000 DE 20018772 U; 13.03.2001 EP 01106091
(43) Veröffentlichungstag der Anmeldung: 08.05.2002
(73) Patentinhaber: Toussaint, Winfried, Dr., 64625 Bensheim (DE)
(72) Erfinder: Toussaint, Winfried, Dr., 64625 Bensheim (DE)
(74) Vertreter: Benz, Jürgen

(56) Entgegenhaltungen:
- WO-A-95/33418
- DE-A- 19 844 628
- DE-U- 20 018 772
- US-A- 5 462 066

## Beschreibung

Die Erfindung betrifft eine Aufbißschiene zur Verhinderung von Schnarchen und von obstruktiver Schlafapnoe sowie zur Not- und Heimbeatmung.

Eine Aufbissschiene gemäß dem Oberbegriff von Anspruch 1 ist aus der DE-A-19844628 bekannt.

Schnarchen kann ein Symptom sein für nächtliche Atemstillstände, die schwerwiegende gesundheitliche Komplikationen, wie z.B. Herz-Kreislauferkrankungen nach sich ziehen können. Durch das US-PS 5,462,066 ist eine derartige zahnspangenartige Aufbißschiene zur Verhinderung des Schnarchens bekannt geworden, welche dazu dient, den Unterkiefer geringfügig nach vorne zu verschieben, weil in dieser Stellung des Unterkiefers die Atemwege weiter geöffnet werden, so dass der Patient freier atmen kann, ohne zu schnarchen.

Die bekannte Aufbißschiene in Form eines zahnspangenartigen Mundstücks besteht aus einem thermoplastischen Material mit zwei Bißrillen, welches mit Erwärmung formbar wird. Der Patient nimmt das erwärmte, noch nicht angepasste Mundstück in den Mund, um dann in den formbaren Kunststoff die Zähne des Unter- sowie des Oberkiefers in die entsprechende untere sowie obere Bißrille hineinzudrücken und durch Aufbeißen auf die Bißplatten der Bißrillen anzupassen. Dabei kühlt der Kunststoff ab und gewinnt seine feste Elastizität zurück, wonach nunmehr das Mundstück an den Patienten angepasst ist. Beim Anpassungsvorgang muß darauf geachtet werden, den Unterkiefer etwas nach vorne zu verschieben. Die bekannte Aufbißschiene besitzt den Nachteil, dass dieselbe im Bereich der übereinander liegenden Bißrillen zu dünn ausgeführt ist, so dass ein Durchbeißen der Bißplatte nach häufigem Benutzen möglich ist. Des weiteren besitzt die bekannte Aufbißschiene keine Notatemluftöffnung, so dass bei Schlafapnoe in Extremfällen sogar eine lebensgefährliche Situation entstehen kann.

Der Erfindung liegt die Aufgabe zugrunde, eine Aufbißschiene zur Verhinderung von Schnarchen sowie von obstruktiver Schlafapnoe zu schaffen, welche gegenüber dem Stand der Technik verbessert ist und insbesondere von der noch nicht angepassten Form ausgehend eine einfache höchst individuelle Anpassung an das Desmodont des Patienten ermöglicht, wobei sowohl die Lebensdauer als auch die Sicherheit der Aufbißschiene verbessert sein soll. Ebenso soll die Erfindung den Anschluß eines Beatmungsgerätes an die Aufbißschiene ermöglichen, um diese auch im klinischen und Home-Care Bereich einzusetzen.

Gelöst wird die Aufgabe durch eine Aufbißschiene gemäß Anspruch 1.

In einer weiteren vorteilhaften Ausgestaltung der Aufbißschiene weist diese im Frontbereich ein schlitzförmiges Luftloch zur Sicherstellung einer Notatmung auf, welches die Aufbißschiene vollständig bis in den Mundhöhlenbereich durchsetzt, wobei das Luftloch länglich-oval oder länglich rechteckförmig gestaltet sein kann und vorzugsweise länglich-oval ist. In einer weiteren Ausgestaltung der Erfindung kann das schlitzförmige Luftloch wenigstens in den Eckzahnbereichen oder zwischen Eckzahnbereich und Schneidezahnbereich beginnen und über die Schneidezahnbereiche hinweglaufen. In einer besonderen Ausgestaltung kann das schlitzförmige Luftloch auch schon im Bereich des ersten Molaren beginnen.

In einer weiteren Ausgestaltung der Aufbißschiene beträgt die Dicke der Bißplatte zwischen dem Boden der Oberkieferbißrille und dem Boden der Unterkieferbißrille zwischen 5 bis 7 mm oder 5 bis 8 mm, vorzugsweise 6 mm. Diese Dicke der horizontalen Bißplatte kann vorzugsweise durchgehend gleichbleibend außerhalb des Bereichs des Luftloches sein.

Aufgrund der Verdickung der horizontalen Bißplatte wird zum einen eine bessere, nämlich tiefere Kieferimpression, erreicht, zum anderen wird dadurch die Stabilität der Aufbißschiene ohne Verlust an Tragekomfort erhöht. Die Erhöhung der Stabilität wird auch zusätzlich durch eine höhere Erweichungstemperatur der Kopolymerenmischung erreicht. Dadurch wird die Lebensdauer der Aufbißschiene, insbesondere bei Patienten mit nächtlichem Zähneknirschen, entscheidend verlängert.

In einer weiteren Ausgestaltung der Aufbißschiene weist dieselbe im Bereich um das schlitzförmige Luftloch eine Verdickung wenigstens der horizontalen Bißplatte der Oberkieferbißrille auf. Vorzugsweise weist auch die horizontale Bißplatte der Unterkieferbißrille um das schlitzförmige Luftloch eine eben solche Verdickung auf. Dadurch ist ein Durchbeißen der Aufbißschiene im Bereich des schlitzförmigen Luftloches, wo ja ansonsten eine Materialschwächung vorliegen würde, praktisch nicht mehr möglich wie auch eine bessere Impression im Bereich der Schneidezähne erreicht wird. Die Verdickung der Bißplatte im Bereich um das Luftloch herum gegenüber dem Bereich außerhalb des Luftloches beträgt zwischen 1mm bis 4mm, vorzugsweise zwischen 1mm bis 2mm.

In einer weiteren Ausgestaltung der Erfindung beginnt die Verdickung der Bißplatte vor dem schlitzförmigen Luftloch im frontseitigen Kurvenbereich der Aufbißschiene, wenigstens im Eckzahnbereich, gegebenenfalls schon im Bereich des ersten Molaren, wobei die Verdickung in praktisch gleichbleibender Stärke über das Luftloch im Bereich der Schneidezähne hinweg läuft und zwar vorzugsweise sowohl im Bereich der Oberkieferbißrille als auch im Bereich der Unterkieferbißrille.

Um das Luftloch herum ist die horizontale Bißplatte auf der Unterseite bzw. auf der dem Unterkiefer zugewandten Seite um je zwischen 1mm bis 4mm, vorzugsweise zwischen 1mm bis 2mm verdickt, um im Frontbereich eine gute Impression der Schneidezähne zu erreichen.

Des weiteren ist der erfindungsgemäßen Aufbißschiene ein Modellierungsstreifen beigelegt, um beispielsweise durch den Zahnarzt bei einem Molarendeckbiß den Hohlraum zwischen den Backenzähnen von Unter- und Oberkiefer individuell ausgleichen zu lassen.

In einer weiteren Ausgestaltung der Aufbißschiene ist die Geometrie der Front- und Seitenflächen zur Erhöhung des Tragekomforts abgeflacht, wobei die Abflachungen auf beiden Seiten zu den Enden der Aufbißschiene hin verlaufen. Die Front- und Seitenflächen werden durch Seitenwangen gebildet, nämlich durch eine rechtsseitige innere und eine linksseitige sowie durch eine rechtsseitige und eine linksseitige äußere Seitenwange. Die rechtsseitige innere bzw. äußere Seitenwange geht im Bereich des Luftloches in die linksseitige innere bzw. äußere Seitenwange über. In einer hierzu weiteren Ausgestaltung der Aufbißschiene ist die Bißleiste an ihrem linksseitigen wie rechtsseitigen molaren Ende verlängert bis unter den letzten Molaren, wobei im Bereich des letzten Backenzahnes die jeweiligen äußeren und inneren Seitenwangen auch geringfügig zurückgesetzt sein können. Die Aufbißschiene mitsamt Bißleiste reicht somit vorzugsweise so weit wie Backenzähne im Kiefer vorhanden sind. Durch diese Verlängerung der horizontalen Bißplatte nach hinten wird eine Auflage auch der hintersten Molaren in der oberen und unteren Zahnreihe erreicht, wodurch eine Entwicklung von theoretisch nicht auszuschließenden Backenzahnfehlstellungen bei langfristigem Gebrauch der Aufbißschiene sicher vermieden wird.

Die erfindungsgemäße Aufbißschiene besitzt den Vorteil, dass die Progenierung des Unterkiefers grundsätzlich durch eine gleichmäßige Impression sämtlicher Zähne erreicht wird. Des weiteren besitzt die Aufbißschiene den Vorteil, dass sie unkompliziert ohne besondere Hilfsmittel innerhalb weniger Minuten angepasst werden kann. Aus diesem Grund genügt auch eine Standardschiene passend für fast sämtliche Kieferformationen. Des weiteren ist in höchst vorteilhafter Weise eine individuelle Einstellung des Unterkiefervorschubs möglich.

Beispiele der Erfindung sind in der Zeichnung dargestellt und anschließend beschrieben, wobei zeigen:
- Figur 1: eine perspektivische Ansicht von schräg oben und hinten der Aufbißschiene zur Darstellung der Oberkieferbißrille
- Figur 2: eine perspektivische Ansicht von vom oben der Aufbißschiene zur Darstellung der Frontansicht
- Figur 3: eine Aufsicht von unten auf die Aufbißschiene zur Darstellung der Unterkieferbißrille
- Figur 4: eine schematische Schnittansicht durch die Aufbißschiene der Figur 1 zur Darstellung der Verdickungen der horizontalen Bißplatte der Unterkiefer- sowie der Oberkieferbißrille
- Figur 5: eine Ansicht von vorne zur Darstellung des schlitzförmigen Luftloches sowie der Verdickung der horizontalen Bißplatte der Oberkieferbißrille sowie der Verdickung der horizontalen Bißplatte der Unterkieferbißrille
- Figur 6: eine Ansicht von vorne einer weiteren Aufbißschiene zur Darstellung eines länglich-ovalen Luftloches sowie der vor dem Luftloch beginnenden Verdickungen der horizontalen Bißplatte der Ober- und Unterkieferbißrille
- Figur 7: eine Ansicht von vorne einer weiteren, ähnlichen Aufbißschiene zur Darstellung eines länglich-rechteckigen Luftloches sowie der vor dem Luftloch beginnenden Verdickungen der horizontalen Bißplatte der Ober- und Unterkieferbißrille
- Figur 8: eine Draufsicht auf die Aufbißschiene der Figur 6 zur Darstellung der Verlängerungen des linksseitigen und rechtsseitigen Endes der Bißleisten ohne Seitenwangen
- Figur 9: eine perspektivische Explosionsdarstellung einer Aufbißschiene der Figur 6 mit einem Anschluß an ein Beatmungsgerät, bestehend aus einer Tülle, dessen der Aufbißschiene zugewandtes Ende der frontseitigen Öffnung des Luftloches angepaßt ist und auf die Tülle ein Verbindungsschlauch anschließbar ist und
- Figur 10: die Aufbißschiene der Figur 9 in ihrem integrierten Zustand nach der Herstellung.

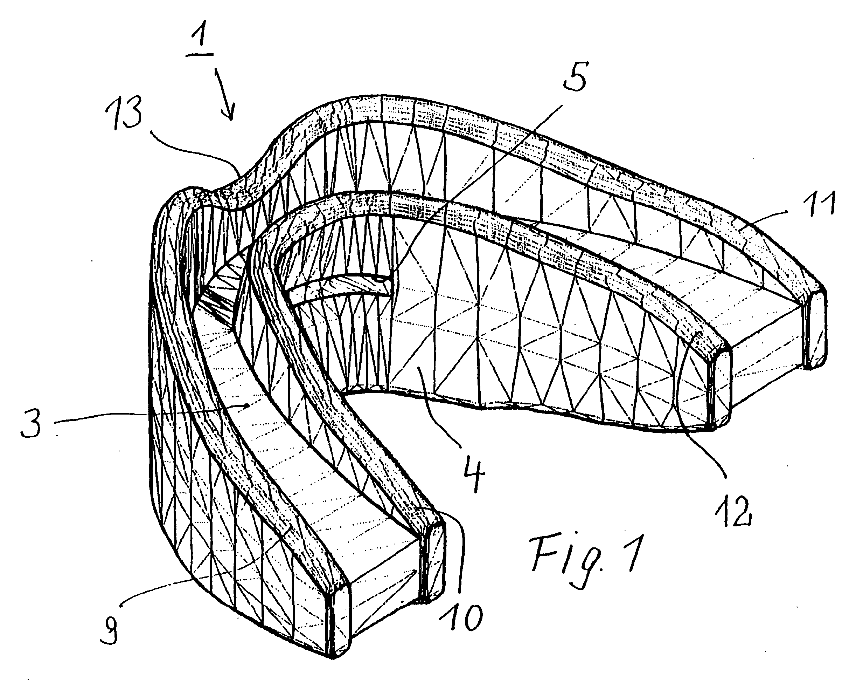

Ein Beispiel einer Aufbißschiene gemäß der Erfindung zur Verhinderung von Schnarchen sowie von obstruktiver Schlafapnoe ist in Figur 1 abgebildet, wobei die Aufbißschiene aus einem Gemisch zweier thermoplastischer Kopolymere besteht. Jedes Kopolymer besteht seinerseits aus einem Gemisch von Polyethylen und Polyvinylacetat. Zur Herstellung der Aufbißschiene werden 50 Gewichtsprozent des ersten thermoplastischen Kopolymers sowie 50 Gewichtsprozent des zweiten thermoplastischen Kopolymers, welche beispielsweise beide in Granulatform vorliegen, gemischt.

Um eine Erweichungstemperatur des resultierenden Kopolymers zu erreichen, die über der körpereigenen Temperatur liegt, jedoch ohne das Mundgewebe zu verbrennen, muß der Vinylacetatgehalt in den beiden thermoplastischen Kopolymeren zwischen 25 und 33 Gewichtsprozent liegen. Die Erweichungstemperatur des ersten Kopolymers ist so gewählt, dass sie bei 40 C° liegt, die Erweichungstemperatur des zweiten Kopolymers ist derart gewählt, dass sie bei 46 C° liegt.

Wenn die beiden Kopolymere im Mischbehälter der Spritzgussmaschiene im Gewichtsverhältnis 1:1 oder nahezu 1:1 bzw. mehr oder weniger 1:1 gemischt werden, so liegt die resultierende Erweichungstemperatur der endgültigen Mischung bz. der Aufbißschiene zwischen 43 bis 46 C°, vorzugsweise bei 44 Grad Celsius.

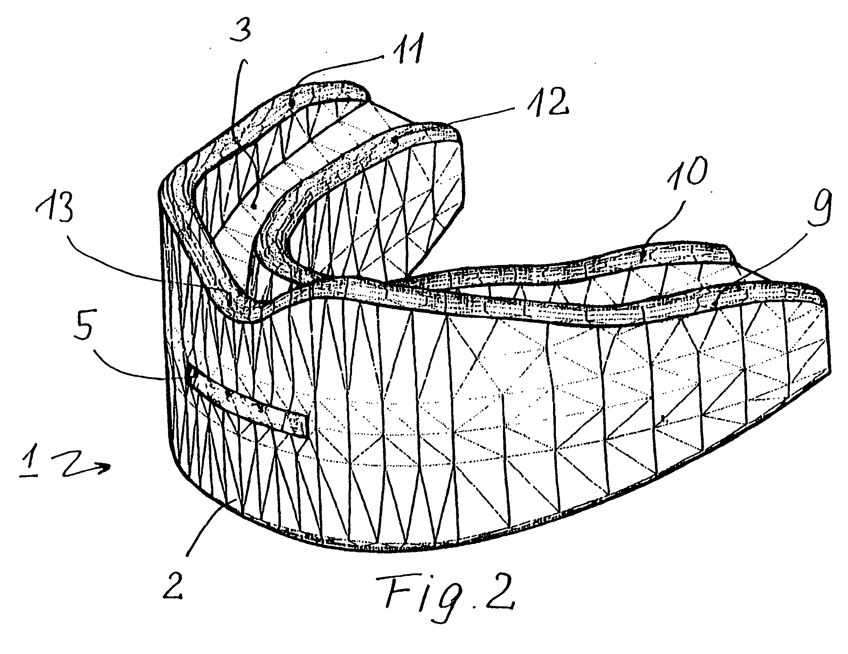

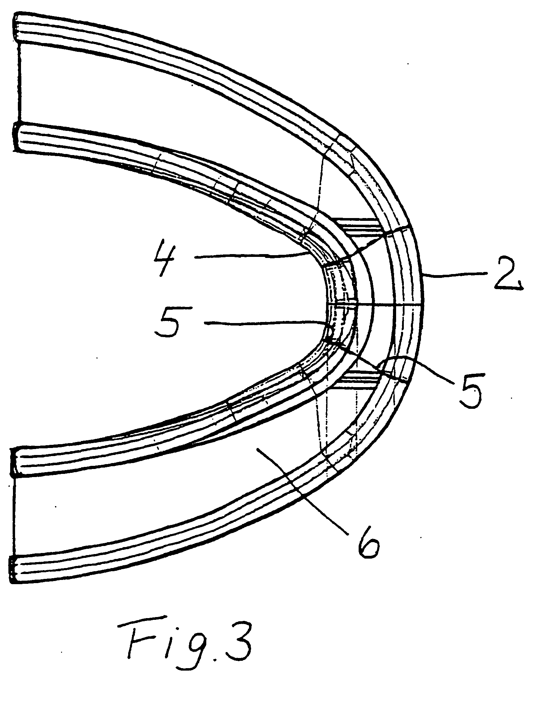

Die Aufbißschiene 1 der Figuren besteht aus einem zahnspangenförmigen Körper, welcher entsprechend dem menschlichen Ober- und Unterkiefer gekrümmt ist. Die Aufbißschiene 1 besteht aus einem Material aus thermoplastischen Kunststoffgemisch, wobei die Aufbißschiene 1 dem menschlichen Ober- und Unterkiefer nachgeformt ist und in das Material der Aufbißschiene 1 im noch nicht angepassten Zustand zur Aufnahme der Zähne eine Oberkieferbißrille 3, Figur 2, und eine Unterkieferbißrille 6, Figur 3, eingeformt ist.

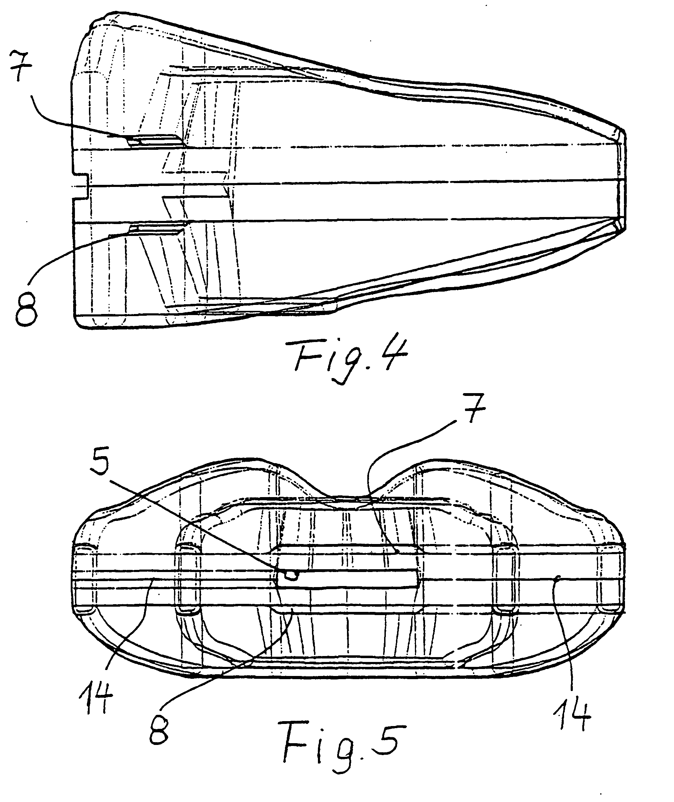

Die Aufbißschiene 1 weist eine nach außen gewandte Frontseite 2 sowie eine nach innen, der Zunge zugewandte Rückseite 4 auf. Die Aufbißschiene 1 läuft nach hinten, das ist zum Inneren der Mundhöhle, abgeflacht aus und besitzt linksseitige innere und äußere Seitenflächen bzw. Seitenwangen 9, 10 sowie rechtsseitige innere und äußere Seitenflächen bzw. Seitenwangen 11,12. welche in ihrer Dicke, Vertikalausedehnung, abnehmen, wie es den Figuren 1 und 2 zu entnehmen ist. Die Kanten der Seitenwangen 9, 10, 11, 12 sind vorzugsweise abgerundet, insbesondere im Bereich der hinteren Enden der Seitenwangen. Die Frontseite 2 (Figur 2) besitzt im oberen Bereich eine Einbuchtung 13 für den oberen Lippenbändchenbereich. In der Mitte wird die Aufbißschiene 1, ausgehend von der Frontseite 2 bis zur Rückseite 4, durch ein waagrecht verlaufendes, schlitzförmiges Luftloch 5 durchsetzt, welches somit die horizontal verlaufende Bißplatte 14, begrenzt durch den Boden der Oberkieferbißrille 3 und den Boden der Unterkieferbißrille 6, durchsetzt, wobei sich das schlitzförmige Luftloch 5 im Bereich der Schneidezähne befindet. Aus den Figuren 4 und 5 ist zu erkennen, dass die horizontale Bißplatte 14 der Oberkieferbißrille 3 im Bereich des Luftloches 5 eine Verdickung 7 von ca. 1mm aufweist; ebenso weist die horizontale Bißplatte 14 der Unterkieferbißrille 6 im Bereich des Luftloches 5 eine Verdickung 8 von cirka 1mm bis 2mm, vorzugsweise 1,5mm, auf, wie es insbesondere der Figur 5 zu entnehmen ist. Auf diese Weise wird im Bereich des schlitzförmigen Luftloches, 5 die Materialwandung sowohl der Bißplatte 14 der Oberkieferbißrille 3 wie auch die Materialwandung der Bißplatte 14 der Unterkieferbißrille 6 im Bereich des schlitzförmigen Luftloches 5 verdickt und verstärkt. Die horizontale Bißplatte 14 ist somit im Bereich des Luftloches 5 sowohl nach oben als auch nach unten verdickt.

Des weiteren ist es möglich, die erfindungsgemäße Aufbißschiene in mehrfachen Erweichungs- und Abkühlungsvorgängen mehrfach anzupassen, falls die optimale Kieferposition beim ersten Anpassungsvorgang nicht erreicht wird. Des Weiteren ist den Figuren 1 und 4 zu entnehmen, das die Geometrie der Frontseite 2 hin zu den Seitenflächen 9, 10, 11 und 12 sowie diese Seitenflächen selbst abgeflacht sind. Die Abflachung ist dabei mehr oder weniger geradlinig bzw. kontinuierlich abfallend zu den Enden der Aufbißschiene ausgebildet und dem Kieferverlauf angepasst. Auf diese Weise wird eine Verbesserung des Tragekomforts der Aufbißschiene erreicht.

Ein weiterer Vorteil der erfindungsgemäßen Aufbißschiene 1 besteht darin, dass diese in weitaus den meisten Fällen keine Anpassung durch Schneiden von Material der Seitenflächen 9, 10, 11 und 12 oder der Frontfläche 2 benötigt. Die Aufbißschiene 1 ist als Standardschiene ausgeführt, welche für fast sämtliche Kieferformationen passt und deshalb in aller Regel keinen individuellen Zuschnitt benötigt.

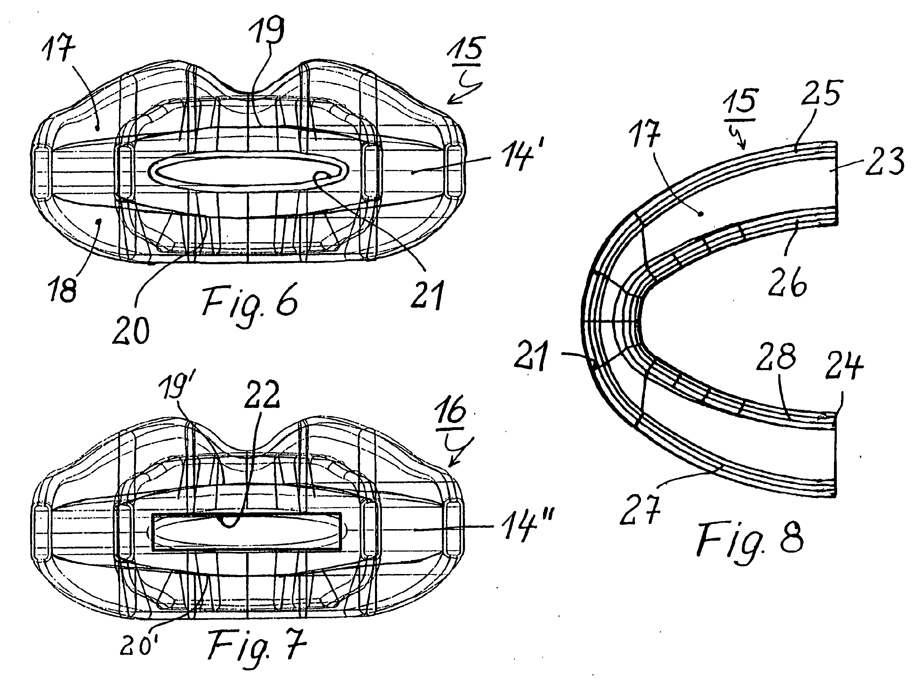

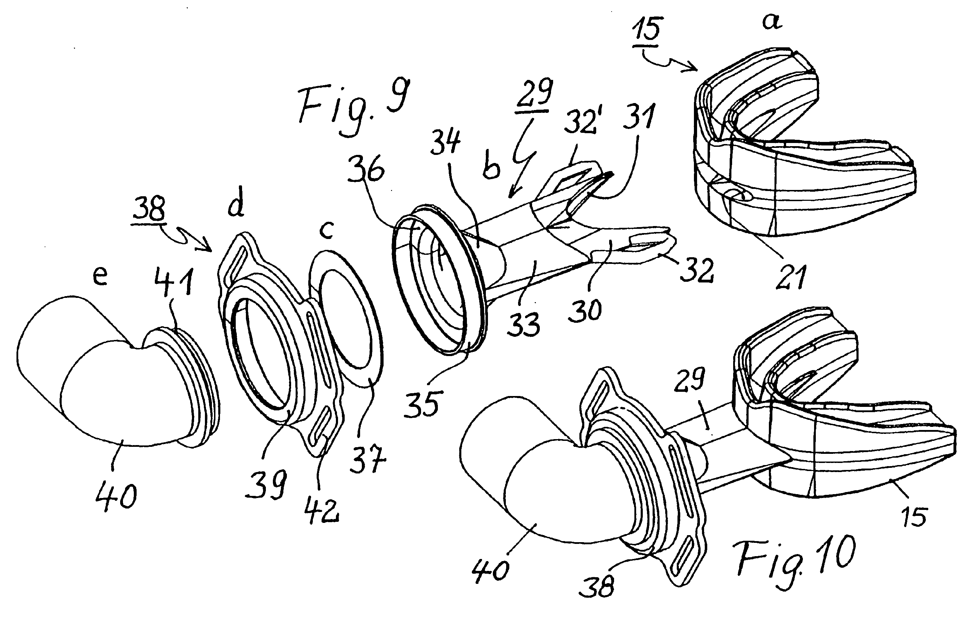

Die Figuren 6, 7 und 8 zeigen zwei weitere Ausgestaltungen zweier Aufbißschienen 15 und 16 in Frontansicht, welche ähnlich der Aufbißschiene 1 der Figur 1 gestaltet ist mit Oberkieferbißrille 17 und Unterkieferbißrille 18 sowie Bißplatte 14' bzw. 14". Die Figur 8 zeigt eine Draufsicht auf Figur 6, wobei mit den Bezugsziffern 25, 26 die äußere und innere linksseitige Seitenwange im molaren Bereich, mit den Bezugsziffern 27, 28 die äußere und innere rechtsseitige Seitenwange im molaren Bereich bezeichnet sind. An ihren molaren Enden weist die Bißschiene Verlängerungen 23, 24 auf, welche zwischen 3mm bis 7mm, vorzugsweise 4mm, betragen, so dass die Bißschiene bis unter den letzten Molaren in der oberen und unteren Zahnreihe reicht.

Die Aufbißschienen 15, 16 besitzen größere, durchgehende Luftlöcher 21, 22 im frontalen Bereich, wobei das Luftloch 21 der Aufbißschiene 15 länglich-oval und das Luftloch 22 der Aufbißschiene 16 länglich-recheckförmig geformt ist. Des weiteren beginnt hier jeweils die Verdickung 19, 20 bzw. 19', 20' der Bißplatte 14' bzw. 14" vor dem schlitzförmigen Luftloch 21, 22 im frontseitigen Kurvenbereich der Aufbißschiene 15, 16, was den Figuren 6 und 7 zu entnehmen ist. Die Verdickung 19, 20 bzw. 19', 20' der Bißplatte 14' bzw. 14" beginnt vorzugsweise wenigstens im Eckzahnbereich, gegebenenfalls schon im Bereich des ersten Molaren; die Verdickung 19, 20 bzw. 19', 20' läuft in gleichbleibender Stärke über das Luftloch 21, 22 im Bereich der Schneidezähne hinweg und zwar vorzugsweise sowohl im Bereich der Oberkieferbißrille 17 als auch im Bereich der Unterkieferbißrille 18. Diese Verdickungen betragen oben und unten zwischen 1mm bis 5mm, vorzugsweise zwischen 2,5mm bis 3,5mm. Sie verlaufen zu den hinteren Seitenbereichen abflachend bis auf das Niveau der horizontalen Bißplatte 14', 14", welche vorzugsweise eine Dicke von 4mm aufweist. Die Luftlöcher haben eine bevorzugte Größe von maximal 4mm Höhe und einem Innendurchmesser von 27mm.

Die Figur 9 zeigt eine perspektivische Explosionsdarstellung einer Aufbißschiene der Figur 6, bestehend aus einem Material aus thermoplastischen Kunststoffgemisch, wobei die Aufbißschiene zahnspangenartig dem menschlichen Ober- und Unterkiefer nachgeformt ist und in das Material der Aufbißschiene zur Aufnahme der Zähne eine Oberkieferbißrille und eine Unterkieferbißrille eingeformt sind. Die Aufbißschiene, welche zur Sicherstellung einer Notatmung oder Heimbeatmung einen Anschluß an ein Beatmungsgerät besitzt, besteht aus einer Tülle, dessen der Aufbißschiene zugewandtes Ende der frontseitigen Öffnung des Luftloches angepaßt ist und auf die Tülle ein Verbindungsschlauch anschließbar ist zum Anschluß des Beatmungsgerätes; die Figur 10 zeigt die Aufbißschiene der Figur 9 in zusammengebautem Zustand.

Gemäß den Figuren 9 und 10 weist die Aufbißschiene 15 ein im Frontbereich angeordnetes, schlitzförmiges Luftloch 21 zur Sicherstellung einer Not- oder Heimbeatmung auf, welches die Aufbißschiene 15 vollständig bis in den Mundhöhlenbereich durchsetzt, wobei das Luftloch 21 länglich-oval gestaltet ist. Das Luftloch 21 ist luftdicht mit einem Verbindungsstück 29 von außen verbunden, welches in das Luftloch 21 eingesetzt ist, wobei auf das Verbindungsstück 29 Anschlußteile 29, 40 zum Anschluß eines (nicht dargestellten) Beatmungsgerätes aufgesetzt sind.

Das Verbindungsstück 29 besteht im Wesentlichen aus einer rohrförmigen Flachtülle 33, welche ein der Aufbißschiene 15 zugewandtes und ebenfalls flach geformtes Endstück 30 aufweist, welches seinerseits eine ovale Aussparung 31 besitzt. Seitlich weist das Endstück 30 des Verbindungsstücks 29 zwei Federbügel 32, 32' auf, welche zur Verankerung innerhalb der Bißplatte 14, 14', 14" dienen. Hierzu besteht das Endstück 30 des Verbindungsstücks 29 vorzugsweise aus demselben Material wie die Aufbißschine 15 selbst, nämlich aus Polyethylenvynilacetat (PEVA). Bei der Herstellung wird das Endstück 30 mit den Federbügeln 32, 32' in der Form für die Aufbißschiene 15 plaziert und beim Spritzvorgang werden die Federbügel 32, 32' in die Bißplatte 14, 14', 14" eingespritzt, womit das Verbindungsstück 29 fest an der Aufbißschine verankert ist und Aufbißschiene sowie Verbindungsstück 29 ein integrales Teil bilden.

An ihrem der Aufbißschiene 15 entgegengesetzten Ende besitzt das Verbindungsstück 29 einen kegelförmigen Übergang 34 mit einer sich daran anschließenden Ringwandung 35, wobei innen unterhalb der Ringwandung 35 ein Innenflansch 36 zur Auflage eines Dichtrings 37 angeordnet ist. Auf oder in die Ringwandung 35 des Verbindungsstücks 29 ist ein Adapter 38 mit einem Aufnahmering 39 aufgesetzt, in welchen ein rohrförmiges Winkelstück 40 mit einem Steckflansch 41 eingesteckt ist, wobei an das andere Ende des Winkelstücks 40 ein Beatmungsgerät anschließbar ist. Der Adapter weist Haltelaschen 42 auf zur Befestigung einer (nicht dargestellten) Kopfhaube.

Das Verbindungsstück 29, mit Ausnahme des Endstücks 30, der Adapter 38 wie auch das Winkelstück 40 bestehen aus einem festen und harten Material, zum Beispiel aus Polycarbonat.

### Gewerbliche Anwendbarkeit:

Die Erfindung ist gewerblich anwendbar insbesondere im Bereich des Gesundheitswesens, der Medizintechnik, der Zahnmedizin sowie der Home-Care Versorgung. Wenn die Aufbißschiene von außen mit einem Verbindungsstück auf dem Luftloch verbunden ist, kann an das Verbindungsstück ein Anschlußteil zum Anschluß eines Beatmungsgerätes aufgesetzt werden, so dass die Aufbißschiene vorteilhaft auch im klinischen Bereich zur Notbeatmung wie auch zur Heimbeatmung, zum Beispiel bei obstruktiver Schlafapnoe, über den Mund, auch zur Überdruckbeatmung, eingesetzt werden kann.

### Liste der Bezugszeichen:

- 1, 15, 16: Aufbißschiene
- 2: Frontseite
- 3,17: Oberkieferbißrille
- 4: Rückseite
- 5, 21, 22: schlitzförmiges Luftloch
- 6, 18: Unterkieferbißrille
- 7, 19, 19': Verdickung der horizontalen Bißplatte der Oberkieferbißrille
- 8, 20, 20': Verdickung der horizontalen Bißplatte der Unterkieferbißrille
- 9,10, 25, 26: äußere, innere linksseitige Seitenwange im molaren Bereich
- 11, 12, 27,28: äußere, innere rechtsseitige Seitenwange im molaren Bereich
- 13: Einbuchtung
- 14, 14',14": horizontale Bißplatte
- 23, 24: Verlängerungen des linksseitigen und rechtsseitigen Endes der Bißleisten mit Seitenwangen
- 29: Verbindungsstück
- 30: flaches Endstück des Verbindungsstücks
- 31: ovale Aussparung des Endstücks des Verbindungsstücks
- 32, 32': Federbügel
- 33: Flachtülle
- 34: kegelförmiger Übergang
- 35: Ringwandung
- 36: Innenflansch
- 37: Dichtring
- 38: Adapter
- 39: Aufnahmering
- 40: rohrförmiges Winkelstück
- 41: Steckflansch
- 42: Haltelaschen

## Patentansprüche

1. Aufbissschiene (1, 15, 16) zur Verhinderung von Schnarchen und von obstruktiver Schlafapnoe, bestehend aus einem thermoplastischem Kunststoffmaterial, wobei die Aufbissschiene (1, 15, 16) zahnspangenartig dem menschlichen Ober- und Unterkiefer nachgeformt ist und in das Material der Aufbissschiene (1,15,16) zur Aufnahme der Zähne eine Oberkieferbissrille (3, 17) und eine Unterkieferbissrille (6,18) eingeformt ist, **dadurch gekennzeichnet, dass** das thermoplastische Kunststoffmaterial eine Mischung aus zwei thermoplastischen Kopolymeren ist, welche beide ihrerseits aus Polyethylen und Polyvinylacetat mit einem Gehalt an Vinylacetat zwischen 25 und 33 Gewichtsprozent bestehen, und die Erweichungstemperatur des einen Kopolymers bei 40 Grad Celsius und die des anderen bei 46 Grad Celsius gewählt ist, so dass die Erweichungstemperatur des Kunststoffgemisches zwischen 43, und 45 Grad Celsius liegt.

2. Aufbissschiene gemäß Anspruch 1, **dadurch gekennzeichnet, dass** die zwei thermoplastischen Kopolymeren im Verhältnis 1 : 1 vorliegen.

3. Aufbissschiene gemäß Anspruch 1 oder 2, **dadurch gekennzeichnet, dass** die Erweichungstemperatur des Kunststoffgemisches bei 44 Grad Celsius liegt.

4. Aufbissschiene nach einem der Ansprüche 1- 3, **dadurch gekennzeichnet, dass** die Geometrie der Front- und Seitenwangen (9, 10, 11, 12, 25, 26, 27, 28) zur Erhöhung des Tragekomforts zu den molaren Enden der Aufbissschiene (1,15,16,23,24) hin abgeflacht verläuft.

5. Aufbissschiene nach einem der Ansprüche 1 - 4, **dadurch gekennzeichnet, dass** die Dicke der horizontalen Bissplatte (14) zwischen dem Boden der Oberkieferbissrille (3) und dem Boden der Unterkieferbissrille (6) 4 bis 8 mm beträgt.

6. Aufbissschiene gemäß Anspruch 5, **dadurch gekennzeichnet, dass** die Dicke der horizontalen Bissplatte (14) zwischen dem Boden der Oberkieferbissrille (3) und dem Boden der Unterkieferbissrille (6) 5 bis 7 mm beträgt.

7. Aufbissschiene nach Anspruch 5 oder 6, **dadurch gekennzeichnet, dass** die Dicke der horizontalen Bissplatte (14) zwischen dem Boden der Oberkieferbissrille (3) und dem Boden der Unterkieferbissrille (6) 6 mm, beträgt.

8. Aufbissschiene nach einem der Ansprüch 1 - 7, **dadurch gekennzeichnet, dass** sie im Frontbereich ein schlitzförmiges Luftloch (5, 21, 22) zur Sicherstellung einer Notatmung aufweist, welches die Aufbissschiene (1,15,16) vollständig bis in den Mundhöhlenbereich durchsetzt.

9. Aufbissschiene nach Anspruch 8, **dadurch gekennzeichnet, dass** das Luftloch länglich-oval oder länglich-rechteckförmig gestaltet ist, waagrecht verläuft und eine Höhe von maximal 4 mm und eine Breite von maximal 27 mm aufweist.

10. Aufbissschiene nach einem der Ansprüche 1 - 9, **dadurch gekennzeichnet, dass** sie eine Verdickung (7, 8, 19, 19', 20, 20') der horizontalen Bissplatte (14) der Unterkieferbissrille (6, 18) und / oder der Oberkieferbissrille (3, 17) im Bereich um das schlitzförmige Luftloch (5, 21, 22) herum aufweist.

11. Aufbissschiene nach Anspruch 10 , **dadurch gekennzeichnet, dass** besagte Verdickung (7, 8, 19, 19', 20, 20') der Bissplatte (14) zwischen 1 mm und 5mm beträgt.

## Claims

1. Bite splint (1, 15, 16) for preventing snoring and obstructive sleep apnoea, consisting of a thermoplastic material, where the bite splint (1, 15, 16) is shaped to match the upper and lower human jaw in the manner of a dental brace, and an upper jaw bite channel (3, 17) and a lower jaw bite channel (6, 18) are shaped into the material of the bite splint (1, 15, 16) for accommodating the teeth, **characterized in that** the thermoplastic material is a mixture of two thermoplastic copolymers, both of which themselves consist of polyethylene and polyvinyl acetate having a vinyl acetate content of between 25 and 33 per cent by weight, and the softening point of the first copolymer is selected to be 40 degrees Celsius and that of the other is selected to be 46 degrees Celsius, so that the softening point of the plastic mixture is between 43 and 45 degrees Celsius.

2. Bite splint according to Claim 1, **characterized in that** the two thermoplastic copolymers are present in the ratio 1:1.

3. Bite splint according to Claim 1 or 2, **characterized in that** the softening point of the plastic mixture is 44 degrees Celsius.

4. Bite splint according to one of Claims 1 - 3, **characterized in that** the geometry of the front and side walls (9, 10, 11, 12, 25, 26, 27, 28) flattens out towards the molar ends of the bite splint (1, 15, 16, 23, 24) in order to increase the wearing comfort.

5. Bite splint according to one of Claims 1 - 4, **characterized in that** the thickness of the horizontal bite plate (14) between the base of the upper jaw bite channel (3) and the base of the lower jaw bite channel (6) is 4 to 8 mm.

6. Bite splint according to Claim 5, **characterized in that** the thickness of the horizontal bite plate (14) between the base of the upper jaw bite channel (3) and the base of the lower jaw bite channel (6) is 5 to 7 mm.

7. Bite splint according to Claim 5 or 6, **characterized in that** the thickness of the horizontal bite plate (14) between the base of the upper jaw bite channel (3) and the base of the lower jaw bite channel (6) is 6 mm.

8. Bite splint according to one of Claims 1 -7, **characterized in that** it has in the front region a slot-shaped air hole (5, 21, 22), which passes completely through the bite splint (1, 15, 16) into the oral cavity region, for guaranteeing emergency breathing.

9. Bite splint according to Claim 8, **characterized in that** the air hole has an elongated oval or elongated rectangular shape, runs horizontally and has a maximum height of 4 mm and a maximum width of 27 mm.

10. Bite splint according to one of Claims 1 - 9, **characterized in that** it has a thickening (7, 8, 19, 19', 20, 20') of the horizontal bite plate (14) of the lower jaw bite channel (6, 18) and/or of the upper jaw bite channel (3, 17) in the region around the slot-shaped air hole (5, 21, 22).

11. Bite splint according to Claim 10, **characterized in that** the said thickening (7, 8, 19, 19', 20, 20') of the bite plate (14) is between 1 mm and 5 mm.

## Revendications

1. Gouttière occlusale (1, 15, 16) pour empêcher le ronflement et l'apnée obstructive du sommeil, constituée par un matériau thermoplastique, où la gouttière occlusale (1, 15, 16) est conformée de manière à correspondre aux mâchoires supérieure et inférieure de l'être humain à la manière d'un dispositif de prise d'empreinte dentaire, et un canal de gorge de mâchoire supérieure (3, 17) et un canal de gorge de mâchoire inférieure (6, 18) sont conformés dans le matériau de la gouttière occlusale (1, 15, 16) pour loger les dents, **caractérisée en ce que** le matériau thermoplastique est un mélange de deux copolymères thermoplastiques dont les deux sont eux-mêmes constitués par du polyéthylène et par de l'acétate de polyvinyle présentant une teneur en acétate de vinyle entre 25 et 33 pour cent en poids et le point de ramollissement du premier copolymère est choisi de manière à valoir 40 degrés Celsius et celui de l'autre est choisi de manière à valoir 46degrés Celsius de telle sorte que le point de ramollissement du mélange plastique se situe entre 43 et 45 degrés Celsius.

2. Gouttière occlusale selon la revendication 1, **caractérisée en ce que** les deux copolymères thermoplastiques sont présents selon le rapport 1:1.

3. Gouttière occlusale selon la revendication 1 ou 2, **caractérisée en ce que** le point de ramollissement du mélange plastique est de 44 degrés Celsius.

4. Gouttière occlusale selon l'une des revendications 1 à 3, **caractérisée en ce que** la géométrie des parois avant et latérales (9, 10, 11, 12, 25, 26, 27, 28) s'aplanit en direction des extrémités de molaire de la gouttière occlusale (1, 15, 16, 23, 24) afin d'augmenter le confort de port.

5. Gouttière occlusale selon l'une des revendications 1 à 4, **caractérisée en ce que** l'épaisseur de la plaque de gorge horizontale (14) entre la base du canal de gorge de mâchoire supérieure (3) et la base du canal de gorge de mâchoire inférieure (6) est comprise entre 4 mm et 8 mm.

6. Gouttière occlusale selon la revendication 5, **caractérisée en ce que** l'épaisseur de la plaque de gorge horizontale (14) entre la base du canal de gorge de mâchoire supérieure (3) et la base du canal de gorge de mâchoire inférieure (6) est comprise entre 5 mm et 7 mm.

7. Gouttière occlusale selon la revendication 5 ou 6, **caractérisée en ce que** l'épaisseur de la plaque de gorge horizontale (14) entre la base du canal de gorge de mâchoire supérieure (3) et la base du canal de gorge de mâchoire inférieure (6) est de 6 mm.

8. Gouttière occlusale selon l'une des revendications 1 à 7, **caractérisée en ce qu'**elle comporte, dans la région avant, un trou d'air en forme de fente (5, 21, 22) qui passe complètement au travers de la gouttière occlusale (1, 15, 16) à l'intérieur de la région de la cavité buccale, pour garantir une respiration d'urgence.

9. Gouttière occlusale selon la revendication 8, **caractérisée en ce que** le trou d'air présente une forme ovale allongée ou rectangulaire allongée, il court horizontalement et il présente une hauteur maximum de 4 mm et une largeur maximum de 27 mm.

10. Gouttière occlusale selon l'une des revendications 1 à 9, **caractérisée en ce qu'**elle présente un épaississement (7, 8, 19, 19', 20, 20') de la plaque à gorge horizontale (14) du canal de gorge de mâchoire inférieure (6, 18) et/ou du canal de gorge de mâchoire supérieure (3, 17) dans la région au voisinage du trou d'air en forme de fente (5, 21, 22).

11. Gouttière occlusale selon la revendication 10, **caractérisée en ce que** ledit épaississement (7, 8, 19, 19', 20, 20') de la plaque à gorge (14) est entre 1 mm et 5 mm.
